Europäisches Patentamt

European Patent Office

Office européen des brevets

(19)

(11) Publication number: **0 346 119**
**A2**

# EUROPEAN PATENT APPLICATION

(12)

(21) Application number: **89305792.7**

(22) Date of filing: **08.06.89**

(51) Int. Cl.⁴: **G 01 N 33/563**
**G 01 N 33/569**
**// G01N33/543**

(30) Priority: **08.06.88 US 203730**

(43) Date of publication of application:
**13.12.89 Bulletin 89/50**

(84) Designated Contracting States:
**BE CH DE ES FR GB IT LI NL SE**

(71) Applicant: **CAMBRIDGE BIOSCIENCE CORPORATION**
**365 Plantation Street Biotechnology Research Park**
**Worcester Massachusetts 01605 (US)**

(72) Inventor: **Hung, Chung-Ho**
**12 Windsor Road**
**Milford Massachusetts 01757 (US)**

**Marciani, Dante John**
**48 School Street**
**Hopkington Massachusetts 01748 (US)**

(74) Representative: **Sheard, Andrew Gregory et al**
**Kilburn & Strode 30, John Street**
**London WC1N 2DD (GB)**

(54) Anti-FC assay for detection of antibodies.

(57) This invention is an assay for detecting antibodies in a sample by first capturing, or binding, the antibodies to be detected at the Fc region of the antibodies. In the method of this invention, multiple antibodies may be detected in a single sample. Diagnostic assays involving the use of this method have therefore been developed for the detection of antibodies.

EP 0 346 119 A2

**Description**

## ANTI-FC ASSAY FOR DETECTION OF ANTIBODIES

This invention is in the field of immunoassays, specifically an immunoassay that can detect multiple antibodies in a single sample.

In diagnostic assays for specific antibodies, the detection method typically involves contacting the antibody to be detected with an antigen specific to a domain followed by a specific anti-antibody. Both of these steps involve some degree of non-immunological protein-protein binding. As a result, the assay of these antibodies is characterized by a relatively high background or may even yield a false positive reaction. For example, if an antigen associates non-immunologically with the Fc region of a immunoglobulin G (IgG), it would be difficult to use that antigen to detect the presence of antigen-specific antibodies because it would bind to any IgG and not just to antibodies specifically directed against it. It is desirable to be able to assay such an antigen in a manner which precludes interference from non-immunological affinity for the Fc region.

In recent years, molecules that bind to antibodies through the Fc region have been investigated as immunoglobulin capture reagents. The Fc region of IgG, IgM, and IgA are not involved in the immunological recognition of antigens. Therefore, it is possible to capture antibodies through their Fc region, without steric hindrance of the Fab antigen recognition regions.

Gandhi, B.M., et al., "Enzyme Linked Protein-A: an ELISA for Detection of IgG Antibodies Against Mycobacterium tuberculosis in Intestinal Tuberculosis," Tubercle 67:219-224 (1986), discloses the use of protein A to detect Immunoglobulin G (IgG) antibodies to Mycobacterium tuberculosis in an ELISA assay. In this assay, the microtiter wells were first coated with M. tuberculosis antigen and then serum was added to the antigen-coated wells. Protein A, labeled with the enzyme horseradish peroxidase, was added to detect the antibodies that coupled with the bound antigen.

Sankolli, G.M., et al., "Improvement in the Antibody Binding Characteristics of Microtitre Wells by Pretreatment with Anti-IgG Fc Immunoglobulin, J. Immunol. Methods 104: 191-194 (1987) discloses the use of anti-IgG Fc antibodies to detect estradiol. In this assay, the microtiter wells were first coated with sheep anti-rabbit IgG Fc antibodies and then with rabbit anti-estradiol antibodies. An estradiol-horseradish peroxidase enzyme was used to quantitate the amount of estradiol which bound to the antibodies. According to the results obtained, there was a two-fold improvement in the antigen binding capacity of microtiter wells coated with both anti-IgG Fc antibodies and anti-estradiol antibodies compared with anti-estradiol antibodies alone.

Although there are many assays that are known for detecting antibodies, it would be desirable to develop a sensitive assay that can detect antibodies to multiple antigens in a single sample without steric hindrance of the Fab region and without assay interference due to protein-protein interactions with the Fc region.

The present invention is based on the inventor's realization that a surprising increase in sensitivity and selectivity in some antigen-antibody interactions is obtained when the antibody is presented to the antigen in a manner which shields the Fc region from non-immunological protein-protein interactions and which uniformly orients the antigen recognition sites in a manner which promotes their accessibility to the antigen, and minimizes steric hindrances. These efforts culminated in an assay for detecting specific antibodies in a sample by first binding total serum antibodies at the Fc region of the antibodies. Thus bound, the Fc region is precluded from participating in other protein-protein interactions. In addition, when so bound by its Fc region, the antibody is aligned on a solid surface in an orientation which presents the antigen binding sites in a uniform manner to the antigen being added.

Also in the method of the present invention, the serum or sample may be screened for antibodies against multiple antigens, sequentially or simultaneously, using a single sample. Diagnostic assays involving the use of this method have therefore been developed for the detection of antibodies.

According to the present invention there is provided a method for detecting an antibody in a sample comprising:

(a) capturing an antibody to be detected in a sample at the Fc region of the antibody with an anti-Fc capturing agent;

(b) contacting the captured antibody with a detectably labelled antigen specific to a domain on the antibody to be detected; and

(c) detecting the presence of the antibody.

The present invention further provides a method of detecting multiple antibodies in a sample, which method comprises:

(a) capturing antibodies to be detected in a sample at the Fc region of the antibodies with an anti-Fc capturing agent;

(b) contacting the captured antibodies with a detectably labelled antigen specific to a domain on one of the antibodies to be detected;

(c) contacting the captured antibodies with a second detectably labelled antigen specific to a domain on a second antibody to be detected wherein the second detectably labelled antigen has a different detection label from the first mentioned detectably labelled antigen of step (b); and (d) detecting the presence of the first antibody and the second antibody.

Preferably the method further comprises contacting the captured antibodies with a third detectably labelled antigen specific to a domain on a third antibody to be detected and detecting the presence of the third antibody.

Where the method is for detecting multiple antibodies, the contacting of the captured antibodies with a detectably labelled antigen specific for each of the captured antibodies is done either sequentially or simultaneously.

Preferably, the anti-Fc capturing agent is an anti-Fc antibody, protein A or protein G.

The antibody to be detected is an IgG antibody in some preferred embodiments.

The or each antibody to be detected may be an antibody to a retrovirus, for example HTLV-I, HTLV-II, HIV-1, HIV-2 or STLV.

In some other aspects of the present invention the method is a method of detecting multiple antibodies in a sample comprising:

(a) immobilizing anti-Fc antibodies on a solid support;

(b) contacting the immobilized anti-Fc antibodies with a sample suspected of containing antibodies to be detected such that the antibodies to be detected are captured at their Fc region;

(c) for each of the antibodies to be detected, contacting the captured antibodies with different detectably labelled antigen specific to a domain on the antibody;

(d) detecting the presence of the antibodies by their different labels.

The present invention further provides a kit for the detection of antibodies in a sample, comprising a carrier being compartmentalized to receive one or more container means in close confinement therein, a first container means containing an anti-Fc capturing agent immobilized on a solid phase immunoabsorbant and a second container means containing a first detectably labelled antigen specific to the first antibody to be detected.

Preferably the kit further comprises a third container means containing a second labelled antigen antibody to be detected.

The second container means preferably contains a mixture of detectable labelled antigen to the multiple antibodies to be detected.

This invention comprises an anti-Fc assay for detecting antibodies. By using this assay, there is an unexpected increase in selectivity and sensitivity in the assay. Theoretically and practically, the most efficient way to selectively bind and concentrate specific antibodies is by using their affinity toward the antigen(s) against which they are directed. For instance, affinity chromatography allows the isolation of small amounts of antigen or antibodies by the use of immobilized antibody or antigen, respectively. In enzyme immunoassays, it has been the practice to immobilize antigens or antibodies on solid phases, such as beads, plates, membranes, etc., that can bind or capture antibodies or antigens from the solution surrounding the solid phase. The bound antibody or antigen can be detected by using a second labeled antibody directed against the bound material. This step may add some amplification, due to the fact that more than one molecule of labeled antibody can be bound per molecule of antibody or antigen to be detected. This approach has been used by Gandhi et al., supra, in combination with labeled protein A. Additional sensitivity can be achieved by increasing the concentration of immobilized antigen, favoring the formation of the antigen-antibody complex even in the presence of low antibody concentrations. Therefore, it is expected that an initial selection and concentration by use of the specific affinity is desirable to achieve high sensitivity.

In the Fc capturing immunoassay of the invention, the immunoglobulins are captured through anti-human Fc antibodies without any selection for specificity. In effect, the population bound through the Fc region should represent the immunoglobulin composition of the original sample. Therefore, this approach should not yield any enrichment of the specific immunoglobulins being measured. As a consequence, sensitivity should be lower than that observed with an immobilized antigen procedure. However, as the inventors have surprisingly discovered, the method of the invention utilizing the Fc capturing immunoassay is more sensitive than the antigen binding immunoassay. Serial dilutions of positive serum when tested by the antigen binding procedure show a fast reduction in signal, reflecting the systematic decrease in binding of the antigen specific immunoglobulins. The same serum serial dilutions when tested by the method of the invention show a high signal, or sensitivity, that was maintained over a wide range of serum dilutions.

In addition to increasing the sensitivity of assays for antibodies, the method of this invention provides a quick screening of multiple antibodies. A significant application of this invention is in blood bank screening.

According to the methods of this invention, antibodies that may be used to capture the antibody or antibodies to be tested include antibodies to the immunoglobulin Fc region (Fc antibodies) and other Fc capturing reagents, such as Protein A and Protein G.

In a preferred embodiment of the invention, antibodies to the immunoglobulin Fc region are used to bind the antibodies to be measured to a solid support for subsequent assay and analysis. Polyclonal Fc antibodies are preferred as the Fc capture reagent because of their ability to recognize determinants on classes and subclasses of Fc regions which are not recognized by other proteins such as protein A. However, the present invention also envisions that one of ordinary skill may practice the invention with monoclonal Fc antibodies. One skilled in the art will know how to produce both polyclonal and monoclonal antibodies with specificity for the immunoglobulin Fc region.

The present invention envisions that one of ordinary skill in the art may also use other Fc capture reagents such as protein A or protein G.

Protein A is a cell wall component produced by nearly all strains of Staphylococcus aureus. Protein A binds strongly and specifically to the Fc portion of immunoglobulin IgG, preferring IgG subclasses 1, 2 and 4. Protein A is known and described, for example, in the following documents: European Patent Application, Publication No. 124,374, published 7 November 1984; Uhlen et al., "Complete Sequence of the Staphylococcal Gene Encoding Protein A," J. Biol. Chem. 259:1695-1702 (1984).

Protein G is an Fc receptor found in Group G streptococci. Protein G is well-known in the art and described in numerous publications including PCT Patent Application, Publication No. WO 87/05025; Guss et al., "Structure of the IgG-binding Regions of Streptococcal Protein G," EMB0 J 5:1567-1575 (1986).

Protein G shows a broader range of binding to IgG subclasses than protein A and therefore may be more preferred in a particular application to protein A.

Some HIV-1 antigen-antibody complexes have been characterized by a tendency to produce false positives due to a putative site on the detecting antigen which has an affinity for the Fc region of some IgG antibodies. The practice of this invention effectively eliminates that non-immunological protein-protein interaction from the assay.

Thus, the inventors have recently found that a region of the envelope glycoprotein gp41 of the HIV-I virus interacts in a specific but non-immunological manner with some IgG Fc regions. The AIDS etiological agent, HIV-1, has several polypeptides, one of which (gp160) is highly immunogenic. Antibodies to the gp160 envelope protein, which contains gp41, are the earliest indicators of virus infection. In practice, enzyme immunoassays using gp160 or gp41 regions to capture specific immunoglobulins are being used for screening of blood, patients, etc. The inventors have discovered that a region of gp41 has the unique property of binding some IgGs through their Fc regions. A consequence of this Fc binding is the presence of false-positive results. The binding of IgGs to a gp41 region seems to depend on the structure and type of the IgGs. The mechanism involved in this reaction is currently unknown. Attempts to block this interaction(s) responsible for false-positives, by use of a variety of proteins or sugars, have been unsuccessful. However, use of the Fc capture procedure has completely eliminated this problem of false positives. This Fc binding region in the HIV-1 gp41 is new; other viral protein(s) with this property are currently not known. Nevertheless, it is possible to speculate that related viruses such as HIV-2 may have a similar region, resulting in false positives. The practice of this invention eliminates the non-immunological protein-protein interactions which give rise to these false positives.

The assays of this invention also allow for the detection of multiple antibodies. For example, it is known that acquired immune deficiency syndrome (AIDS) patients may have HIV antibodies directed against the envelope glycoprotein and the core protein. Once the antibodies in a serum sample are captured through their Fc region, antigens specific against each desired antibody may be added to the assay. In the above example, antigens against both the HIV envelope glycoprotein and the core protein could be simultaneously or sequentially added to the captured antibody sample. The antigens may be detectably labeled with the same label, or each antigen may be detected with a different label. Thus, in one embodiment the antibodies can be quantitated together, in an additive manner. In another embodiment, the antibodies may be quantitated together, in a manner which allows for individual identity of each antibody. In yet another embodiment, the antibodies may be quantitated sequentially, using the bound antibody sample in a repetitive manner.

Thus, the advantages of this assay are the increased sensitivity of the assay, the elimination of false positives, and the ability to screen for multiple antibodies.

The method of this invention is not limited to antibodies to HIV. Any antibody capturable by its Fc region may be detected according to this invention. Without being limited, other antibodies that may be detected according to this invention include HIV-2, HTLV-1, hepatitus B, CMV, and EBV.

Samples that may be used in the method of this invention include any that contain reacting IgG, IgM, and IgA antibodies. Typically, in diagnostic tests, a sample containing an unknown amount of antibodies is used, such as urine, saliva, tear drops, cerebrospinal fluid, blood, serum, and the like. Solid or semi-solid samples may also be tested such as tissues, feces, and the like.

The immunoassays within the scope of the present invention include both immunometric assays and competitive assays.

Immunometric assays include forward sandwich, reverse sandwich immunoassays and simultaneous assay. Each of these terms is well understood by those skilled in the art. The immunometric assays will be described for the detection of antibodies to HIV-1. In these assays, the capturing agent to the Fc portion of the antibody is bound to the solid phase carrier and a detection system is used to detect the captured and bound HIV-1 antibodies. This detection system can be a detectably labeled antigen to the antibody to be detected such as that described by Burroughs Wellcome Co., U.S. Patent No. 4,298,685.

In a forward sandwich immunoassay, a sample suspected of containing antibodies against HIV-I is first incubated with a solid phase capturing agent to the Fc portion of the antibody. Incubation is continued for a period of time sufficient to allow the antibodies in the sample to bind to the immobilized Fc capturing agent. After the first incubation, the solid phase is separated from the incubation mixture and washed to remove interfering substances and excess unbound antibody which also may be present in the sample. The solid phase capturing agent with bound antibodies is subsequently incubated for a second time with antigen, preferably labelled in a manner which permits its detection. After the second incubation, another wash is performed to remove unbound, labeled antigen. Labeled antibody bound to the solid phase capturing agent is then measured by the amount of labeled antigen detected. This is a direct measure of the presence of antibodies in the original sample. Forward sandwich assays are described, for example, in United States Patents 3,867,517; 4,012,294; and 4,376,110.

Other known assay systems such as the reverse sandwich assay, the simultaneous sandwich assay, and the delayed immunometric assay may be used in this investigation but are not preferred systems. With each of these assays, the labeled antigen may bind non-specifically to the Fc region of an antibody, resulting in false positive reactions. Capturing the Fc region of the antibody to be detected prior to adding a labeled antigen detection system removes the availability of this region for non-specific binding.

In each of the above assays, the sample-containing antibodies, solid phase capturing agent, and labeled antigen are incubated under conditions and for a period of time sufficient to allow the antibodies to be detected to bind to the immobilized capturing agent and to the labeled antigen. In general, it is desirable to provide incubation conditions sufficient to bind as much sample antibody as possible. Of course, the specific concentrations of labeled antigens, the temperature and time of incubation, as well as other such assay conditions, can be varied, depending upon various factors including the concentration of antibody in the sample, the nature of the sample, and the like. Those skilled in the art will be able to determine operative and optimal assay conditions for each determination by employing routine experimentation.

Further, and importantly, according to this invention, by using the method of this invention, more than one antibody may be detected in a sample. Since the capturing agent will bind to the Fc portion of any antibody, more than one antibody in a sample can be detected by using a different labeling detection system for each antibody to be detected, or, if it is not necessary to individually quantitate each antibody, they may all be assayed together using the same labeling detection system.

The antigen used to detect the antibody or antibodies can be detectably labeled in various ways. The preferred assays for detecting antibodies include radioimmunoassays, enzyme-linked immunosorbent assays (ELISA), or other assays known in the art, such as immunofluorescent assays, chemiluminescent assays, or bioluminescent assays.

Radioactive isotopes which are particularly useful in assays are $^3$H, $^{125}$I, $^{131}$I, $^{32}$P, $^{35}$S, $^{14}$C, $^{51}$Cr, $^{36}$Cl, $^{57}$Co, $^{58}$Co, $^{59}$Fe, $^{75}$Se, and $^{152}$Eu.

While radiolabeling represents one embodiment, alternatively, the anti-antibodies or antigen may also be labeled using fluorescent labels, enzyme labels, free radical labels, avidin-biotin labels, or bacteriophage labels, using techniques known to the art (Chard, Laboratory Techniques in Biology, "An Introduction to Radioimmunoassay and Related Techniques," North Holland Publishing Company (1978).

Typical fluorescent labels include fluorescein isothiocyanate, rhodamine, phycoerythrin, phycocyanin, allophycocyanin, o-phthaldehyde, and fluorescamine.

Typical chemiluminescent compounds include luminol, isoluminol, aromatic acridinium esters, imidazoles, acridinium salts, and the oxalate esters. Typical bioluminescent compounds include luciferin, luciferase, and aequorin.

Typical enzymes include alkaline phosphatase, beta-galactosidase, glucose-6-phosphate dehydrogenase, maleate dehydrogenase, glucose oxidase, and peroxidase.

Two principal types of enzyme assays are enzyme-linked immunosorbent assay (ELISA) and the homogenous enzyme immunoassay, also known as enzyme-multiplied immunoassay (EMIT) (Syva Corp.). The EMIT system depends on deactivation of the enzyme in the tracer-antibody complex; the activity can thus be measured within the need for a separation step.

There are many solid phase immunoabsorbents which have been employed and which can be used in the present invention. Well known immunoabsorbents include beads formed from glass, polystyrene, paper, polypropylene, dextran, nylon, and other material; tubes formed from or coated with such materials, and the like. The immobilized capturing agent may be covalently or physically bound to the solid phase immunoabsorbent, by techniques such as covalent bonding via an amide or ester linkage or by absorption. Those skilled in the art will know many other suitable carriers for binding peptide fragments, or will be able to ascertain such, using routine experimentation.

In addition, the materials for use in the assays of the invention are ideally suited for preparation of a kit. A kit, according to this invention, may comprise a carrier means being compartmentalized to receive in close confinement one or more container means such as vials, test tubes, microtiter wells, plastic dipstick and the like. Each of said container means comprises one of the separate elements to be used in the assay.

In a preferred embodiment of the invention, the materials for use in the assay are provided in an ELISA kit. The present invention also envisions that one of ordinary skill would be able to utilize a kit that provided the materials for use in the invention on a membrane device, dipstick, bead or other solid support configuration such as the ICON system (Hybritech).

For example, one of said container means may comprise the anti-Fc capturing agent immobilized a solid support of a dipstick. A second container means may comprise a test tube for containing the sample with the test antibodies. A third container means may comprise detectably labeled antigen in lyophilized form or in solution that is specific to one of the antibodies to be detected in the sample. A fourth container means may comprise yet another detectably labeled antigen in lyophilized form or in solution that is specific to another of the antibodies to be detected in the sample. Labelled antigens can be assayed separately or mixed together to permit sequential or simultaneous detection.

The carrier may also contain, in addition, a plurality of containers each of which comprises different, predetermined known amounts of antibody. These latter containers can then be used to prepare a standard curve from which can be interpolated the results obtained from the sample containing the unknown amount of antibody.

The following examples are intended to be illustrative, and not limiting.

## EXAMPLES

### Example 1: Comparative Studies for the Sensitivity of αFc-ELISA and Ag-ELISA

Microtiter plates (96-well) were first coated with anti-human IgG Fc immunoglobulins to capture HIV-1 antibodies in human sera. The wells were coated with 200 µl of rabbit anti-human Fc immunoglobulins at 10 µg/ml in 50 mM sodium carbonate, pH 9.6 overnight at room temperature. The plates were then emptied, and a 300 µl volume of 4% BSA in 200 mM sodium phosphate, pH 6.5, was added to each well. After incubation at room temperature overnight, the plates were washed once with phosphate buffered saline, dried under vacuum, and stored at 4°C until use. The ELISA assay was performed as described below. A 190 µl amount of sample diluent was first added to each well coated with the anti-human Fc immunoglobulins. The serum samples to be tested (10 µl) were then added to the wells and incubated at 30°C for 30 minutes. After washing six times with 0.05% Triton X-100 (in water), a 200 µl amount of HIV-I (or HTLV-I) antigen-horseradish peroxidase conjugate at 10 to 100 ng/ml was added to each well. Plates were then incubated at 37°C for one hour and washed six times as before.

To develop the plates, 200 µl of substrate solution (0.032% TMB, 0.025% $H_2O_2$, 50 mM citrate, pH 5.0) was added to each well and incubated at 37°C for 20 minutes. The reaction was stopped by the addition of 100 µl of stopping solution (1 M $H_2SO_4$). The color change in each well was measured spectrophotometrically at 450 nm.

### Ag-ELISA:

Experiments were carried out according to the procedure described for αFc-ELISA with exception that wells were coated with antigen and anti-human Ig conjugate was used instead of antigen-conjugate. Absolute absorbances at 405nM were taken and plotted against serum dilutions.

### Results:

αFc-ELISA is more sensitive than Ag-ELISA. At 24,000-fold serum dilution, a significant amount of reactivity can still be detected by Fc-ELISA. Almost no reactivity can be detected by Ag-ELISA at a dilution of 1:2000. The results are presented in Tables 1 and 2.

Table 1

Comparison of the Reactivity of Sera in αFc-ELISA and Ag-ELISA

| | αFc-ELISA | | | | |
|---|---|---|---|---|---|
| | Ag-Conjugate 2 ng/test | Ag-Conjugate 10 ng/test | Ag-Conjugate 20 ng/test | Ag-ELISA Antigen 100 ng | |
| PL4 | 0.044 | 0.118 | 0.427 | 0.021 | |
| CH450 | 0.039 | 0.167 | 0.601 | 0.021 | |
| 51 | 0.037 | 0.141 | 0.575 | 0.054 | |
| 790 | 0.025 | 0.078 | 0.189 | 0.036 | Negative |
| 97K | 0.029 | 0.109 | 0.227 | 0.029 | |
| 98K | 0.020 | 0.052 | 0.103 | 0.028 | |
| F57 | 0.027 | 0.100 | 0.210 | 0.036 | |
| BBI40 | 0.034 | 0.101 | 0.430 | 1.414 | |
| LA102 | 0.026 | 0.112 | 0.412 | 0.583 | High |
| CHO74 | 0.043 | 0.178 | 0.513 | 0.542 | Background |
| SL410 | 1.467 | >2 | >2 | >2 | |
| 53 | 0.742 | >2 | >2 | 0.773 | |
| 3062 | 1.445 | >2 | >2 | >2 | |
| 3334 | 1.512 | >2 | >2 | >2 | Positive |
| 6965 | 1.493 | >2 | >2 | >2 | |
| 7653 | 1.577 | >2 | >2 | >2 | |
| 7678 | 1.413 | >2 | >2 | >2 | |
| 8005 | 1.353 | >2 | >2 | >2 | |

Absolute absorbance: OD 450 - OD 630

Serum dilution: 1:20

TABLE 2

Comparison of Detection of Antibodies to HIV by Antigen ELISA and αFc ELISA

| Test | Conjugate Conc. | Known Negative | | | False Positives/Pos by Western blot High Background | | | Known Positive | |
|---|---|---|---|---|---|---|---|---|---|
| | | PL4 | MB81073 | MB81503 | BBI40 | LA102 | 823/10 | 3062/19 | RC/200 |
| | | | | | Serum Dilution 1:20 | | | | |
| AgE1A | | 0.018 | 0.025 | 0.056 | 1.619 | 0.767 | 1.027 | >2 | 0.937 |
| αFcEIA | 100 ng/ml | 0.042 | 0.070 | 0.097 | 0.084 | 0.077 | 0.930 | >2 | >2 |
| | 50 ng/ml | 0.100 | 0.037 | 0.056 | 0.097 | 0.050 | 0.753 | >2 | >2 |
| | 10 ng/ml | 0.01 | 0.015 | 0.017 | 0.020 | 0.011 | 0.213 | 0.725 | 0.822 |
| | | | | | Serum Dilution 1:40 | | | | |
| AgEIA | | 0.011 | 0.015 | 0.017 | 1.228 | 0.434 | 0.832 | 1.887 | 0.683 |
| αFcEIA | 100 ng/ml | 0.035 | 0.073 | 0.088 | 0.056 | 0.043 | 0.911 | >2 | >2 |
| | 50 ng/ml | 0.037 | 0.055 | 0.053 | 0.030 | 0.035 | 0.699 | >2 | >2 |
| | 10 ng/ml | 0.08 | 0.012 | 0.015 | 0.010 | 0.011 | 0.20 | 0.766 | 0.804 |
| | | | | | Serum Dilution 1:80 | | | | |
| AgEIA | | 0.016 | 0.016 | 0.016 | 0.807 | 0.222 | 0.551 | 1.483 | 0.459 |
| αFcEIA | 100 ng/ml | 0.056 | 0.077 | 0.097 | 0.064 | 0.035 | 0.974 | >2 | >2 |
| | 50 ng/ml | 0.050 | 0.062 | 0.049 | 0.055 | 0.027 | 0.662 | >2 | 1.856 |
| | 10 ng/ml | 0.010 | 0.014 | 0.025 | 0.013 | 0.014 | 0.190 | 0.808 | 0.545 |

Absolute absorbance: OD 450 - OD630

EP 0 346 119 A2

Example II: Antigen Preparation for αFc-ELISA

Horseradish peroxidase (HRP) conjugates of purified HIV-I or HTLV-I recombinant envelope antigens were prepared by dissolving the antigen in 8 M urea/50 mM borate buffer, pH 9.0, to a concentration of 5 mg/ml. The proteins were succinylated with succinic anhydride at a molar ratio to the lysine residues on the antigens of 1:1. To succinylate the antigens, an 18.9 μl amount of succinic anhydride solution (50 mg/ml in dioxan) was added to 3 ml of the antigen solution. The reaction mixture was allowed to stir at room temperature for 30 minutes while the pH of the solution was maintained at 8.5-9.0 by the addition of 1N NaOH. The solution was then dialyzed extensively against 10 mM sodium carbonate, pM 9.6, and kept at 4°C for conjugation. The succinylated HIV-I and HTLV-I envelope antigens had a degree of succinylation of 30 and 50% of the lysal residues on the antigens, respectively. Immunoreactivity of the antigens succinylated in this way does not differ significantly from that observed with the unmodified antigens. Antigens that were normally soluble, such as gag antigen of both HIV-I and HTLV-I, were used for conjugation without any modifications.

To prepare HRP labelled HIV-I or HTLV-I antigen conjugates, the procedure described by Wilson and Nakane (Wilson, N.B., and Nakane, P.K., (1978) in "Immunofluorescence and Related Techniques," W. Knapp et al., eds., Elsevier Scientific Publishing Co., Amsterdam, pp. 215-225) was followed with some modifications. An amount of 1.16 ml freshly-prepared in 0.1 M NaIO$_4$ was added to 3.8 ml of HRP solution (6 mg/ml in H$_2$O). After stirring at room temperature for 20 minutes, the oxidation reaction was quenched by the addition of 19.4 μl of ethyleneglycol and followed by buffer exchange into 1 mM sodium acetate, pH 4.4, by using a 10 ml Sephadex G-25 column equilibrated with the same buffer. After buffer exchange, 2 mg of succinylated HIV-I antigen (or 1 mg HTLV-I antigen) in 0.645 ml 0.01 M sodium carbonate, pH 9.6, was added to the oxidized HRP solution, and the pH of the solution was raised to 9-9.5 by the addition of 0.4 ml 0.2 M Na$_2$CO$_3$. The reaction was allowed to proceed at room temperature for 2 hours in the dark. The Schiff base formed was then stabilized by the addition of 90 μl of 1 M NaCNBH$_4$ in H$_2$O followed by incubating at 4°C for at least 3 hours. The antigen-HRP conjugates were purified by gel filtration on an S-300 column (2x90 cm) equilibrated and eluted with 0.1 mM Tris-HCl, pH 7.5, at a flow rate of 5 ml/hr. Fractions of 2.5 ml were collected. The desired antigen-HRP conjugates were eluted at the void volume of the column.

The main purpose of the succinylation modification to HTLV-I and HIV-1 was to increase the solubility of otherwise insoluble purified recombinant antigens, although other benefits are achieved as described below.

In general, modification of antigens can diminish the immunoreactivity of the antigens. To overcome this problem, this technique has been developed in which there is modification of only 30-40% of the lysine residues on HIV-I protein and about 50-70% of the lysine residues on HTLV-I antigen. After this limited modification, the antigens become soluble in ordinary buffers and remain immunologically active, as judged by several immunological assays. This technology is particularly useful for antigens that are not soluble in physiological buffers. Examples of insoluble proteins include retrovirus envelope proteins, membrane proteins and hydrophobic proteins.

In addition to increasing the solubility of the antigen, this modification introduces additional negative charges onto the modified antigens. The negatively charged molecules repel each other in solutions, which may result in less aggregation upon prolonged storage of the conjugate in solution. Therefore, this modification should also increase the stability of the conjugates.

Succinylation was chosen because the process is irreversible. This ensures a permanent modification of molecules which is important for long-term stability of the conjugates.

No succinylation is required for soluble antigens such as HIV-I and HTLV-1 gag antigens.

Example III: Fc-ELISA for Two Antibodies

Fc-ELISA assays were conducted to determine whether this assay could detect multiple antibodies in a single human sera sample. The assay procedures are described in Examples I and II. HIV-1 and HTLV-I envelope proteins were used to demonstrate this application, and the results are summarized in Table 3.

HIV-1 (env)-HRP conjugate reacted only with HIV positive sera (column A), and HTLV-I (eny)-HRP conjugate only with HTL-I positive sera (column B). A mixture of HTLV-I (env) conjugate and HIV-1 (env) conjugate reacted with both HTLV-I and HIV-1 positive sera (column C). The absorbance value obtained for the positive serum was not depressed or enhanced by the second conjugate present, suggesting that each antigen-HRP conjugate reacts independently with their specific target antibodies. HIV-1 and HTLV-I coinfected sera (sample DH2827) reacted with all three Fc-ELISA assays: HIV-1 (env) conjugate, HTLV-I (env) conjugate, and the mixture of HIV-1 and HTLV-I. The system is, therefore, very suitable for multiple assays, by either using antigen-HRP conjugates alone or in mixtures.

TABLE 3

αFc-ELISA for Two Antibodies

| | αFc-ELISA | | |
|---|---|---|---|
| | A | B | C |
| | HIV-1 (env)-HRP Conjugate (50 ng/ml) | HTLV-I (env)-HRP Conjugate (100 ng/ml) | HTLV-I (env)-HRP Conjugate (100 ng/ml) + HIV (env)-HRP Conjugate (50 ng/ml) |
| **Negative Sera** | | | |
| PL-4 | 0.027 | 0.021 | 0.041 |
| LA102 | 0.015 | 0.012 | 0.032 |
| CH074 | 0.026 | 0.015 | 0.045 |
| BBI40 | 0.019 | 0.010 | 0.039 |
| **HTLV-I Positive Sera** | | | |
| K14 | 0.026 | 1.588 | 1.552 |
| H9097 | 0.035 | 0.282 | 0.327 |
| H9096 | 0.018 | 0.454 | 0.461 |
| JA163 | 0.026 | 0.441 | 0.485 |
| JA162 | 0.024 | 1.039 | 1.020 |
| JA165 | 0.037 | 1.283 | 1.304 |
| **HIV-1 Positive Sera** | | | |
| 853/10 | 1.170 | 0.023 | 1.338 |
| A10/10 | 0.314 | 0.022 | 0.370 |
| 3334 | >2 | 0.075 | >2 |
| 6965 | >2 | 0.065 | >2 |
| 7673 | >2 | 0.078 | >2 |
| 7678 | >2 | 0.088 | >2 |
| **HIV-1 and HTLV-I Coinfected Sera** | | | |
| DH2827 | >2 | 1.716 | >2 |

Although the foregoing invention has been described by way of illustration and example of purposes of clarity and understanding, it will be obvious that certain changes and modifications may be practiced within the scope of the invention, as limited only by the scope of the appended claims.

**Claims**

1. A method for detecting an antibody in a sample comprising:
(a) capturing an antibody to be detected in a sample at the Fc region of the antibody with an anti-Fc capturing agent;
(b) contacting the captured antibody with a detectably labelled antigen specific to a domain on the antibody to be detected; and
(c) detecting the presence of the antibody.
2. A method for detecting multiple antibodies in a sample, which method comprises:
(a) capturing antibodies to be detected in a sample at the Fc region of the antibodies with an

EP 0 346 119 A2

anti-Fc capturing agent;

(b) contacting the captured antibodies with a detectably labelled antigen specific to a domain on one of the antibodies to be detected;

(c) contacting the captured antibodies with a second detectably labelled antigen specific to a domain on a second antibody to be detected wherein the second detectably labelled antigen has a different detection label from the first mentioned detectably labelled antigen of step (b); and

(d) detecting the presence of the first antibody and the second antibody.

3. A method as claimed in claim 2 further comprising contacting the captured antibodies with a third detectably labelled antigen-specific to a domain on the third antibody to be detected and detecting the presence of the third antibody.

4. A method as claimed in either one of claims 2 or 3 wherein the contacting of the captured antibodies with a detectably labelled antigen specific for each of the captured antibodies is done either sequentially or simultaneously.

5. A method as claimed in any one of claims 1 to 4 therein the anti-Fc capturing agent is an anti-Fc antibody, protein A, or protein G.

6. A method as claimed in any one of claims 1 to 5 wherein the or each antibody to be detected is an IgG antibody.

7. A method as claimed in any one of claims 1 to 6 wherein the or each antibody to be detected is an antibody to a retrovirus, such as HTLV-I, HTLV-II, HIV-1, HIV-2, or STLV.

8. A method as claimed in any of claims 1 to 7 wherein the sample is whole blood, sera or urine.

9. A method of detecting multiple antibodies in a sample comprising:

(a) immobilizing anti-Fc antibodies on a solid support;

(b) contacting the immobilized anti-Fc antibodies with a sample suspected of containing antibodies to be detected such that the antibodies to be detected are captured at their Fc region;

(c) for each of the antibodies to be detected, contacting the captured antibodies with different detectably labelled antigen specific to a domain on the antibody;

(d) detecting the presence of the antibodies by their different labels.

10. A kit for the detection of antibodies in a sample, comprising a carrier being compartmentalized to receive one or more container means in close confinement therein, a first container means containing an anti-Fc capturing agent immobilized on a solid phase immunoabsorbant and a second container means containing a first detectably labelled antigen specific to the first antibody to be detected.

11. A kit as claimed in claim 10 further comprising a third container means containing a second detectably labelled antigen or anti-antibody specific to the second antibody to be detected.

12. A kit as claimed in claim 10 or 11 in which the second container means contains a mixture of detectable labelled antigen to the multiple antibodies to be detected.

11